# EUROPEAN PATENT SPECIFICATION

(11) **EP 3 747 995 B1**
(45) Date of publication and mention of the grant of the patent: **09.11.2022**
(21) Application number: 18897524.7
(22) Date of filing: 25.12.2018
(51) Int. Cl.: A61K 31/704, A61K 35/17, C12N 5/0783, C07K 14/725, A61K 39/00, A61P 35/00

(54) **METHOD FOR PREPARING CHIMERIC ANTIGEN RECEPTOR
(CAR)-CARRYING EXOSOMES DERIVED FROM IMMUNE CELLS, AND USE OF SAID CAR- CARRYING EXOSOMES**
VERFAHREN ZUR HERSTELLUNG VON CHIMÄREN ANTIGENREZEPTOREN
(CAR)-TRAGENDEN EXOSOMEN, DIE VON IMMUNZELLEN STAMMEN, UND VERWENDUNG DIESER CAR-TRAGENDEN EXOSOMEN
PROCEDE DE PREPARATION D'EXOSOMES PORTEURS D'ANTIGENES CHIMERIQUES
(CAR) DÉRIVÉS DE CELLULES IMMUNITAIRES, ET UTILISATION DESDITS EXOSOMES PORTEURS DE CAR

(30) Priority: 26.12.2017 CN 201711432948
(43) Date of publication of application: 09.12.2020
(73) Proprietor: Pharchoice Therapeutics Inc, Shanghai 201406 (CN)
(72) Inventor: HU, Shi, Shanghai 200433 (CN); FU, Wenyan, Shanghai 200433 (CN)
(74) Representative: Petculescu, Ana-Maria
(86) International application number: PCT/CN2018/123298
(87) International publication number: WO 2019/128952

(56) References cited:
- WO-A1-2017/011728
- CN-A- 108 315 305
- BAO HUIJING ET AL: "Chimeric Antigen Receptor-Engineered Exosome As a Drug Delivery System in Mantle Cell Lymphoma", BLOOD, vol. 130, no. Suppl. 1, 7 December 2017 (2017-12-07), page 5561, XP002802783, & 59TH ANNUAL MEETING OF THE AMERICAN-SOCIETY-OF-HEMATOLOGY (ASH); ATLANTA, GA, USA; DECEMBER 09 -12, 2017
- XIANG-JUN TANG ET AL: "Therapeutic potential of CAR-T cell-derived exosomes: a cell-free modality for targeted cancer therapy", ONCOTARGET, vol. 6, no. 42, 29 December 2015 (2015-12-29), XP055585353, DOI: 10.18632/oncotarget.6175
- SANDER A. A. KOOIJMANS ET AL: "Display of GPI-anchored anti-EGFR nanobodies on extracellular vesicles promotes tumour cell targeting", JOURNAL OF EXTRACELLULAR VESICLES, vol. 5, no. 1, 16 January 2016 (2016-01-16), page 31053, XP055416987, ISSN: 2001-3078, DOI: 10.3402/jev.v5.31053
- NICOLAS BLANCHARD ET AL: "TCR activation of human T cells induces the production of exosomes bearing the TCR/CD3/zeta complex.", THE JOURNAL OF IMMUNOLOGY, vol. 168, no. 7, 1 April 2002 (2002-04-01) , pages 3235-3241, XP055057390, ISSN: 0022-1767, DOI: 10.4049/jimmunol.168.7.3235
- JONATHAN M. PITT ET AL: "Dendritic cell-derived exosomes for cancer therapy", THE JOURNAL OF CLINICAL INVESTIGATION, vol. 126, no. 4, 1 April 2016 (2016-04-01) , pages 1224-1232, XP055411136, GB ISSN: 0021-9738, DOI: 10.1172/JCI81137
- WENYAN FU ET AL: "CAR exosomes derived from effector CAR-T cells have potent antitumour effects and low toxicity", NATURE COMMUNICATIONS, vol. 10, no. 1, 25 September 2019 (2019-09-25), XP055705265, DOI: 10.1038/s41467-019-12321-3
- YANG PENGXIANG ET AL: "The exosomes derived from CAR-T cell efficiently target mesothelin and reduce triple-negative breast cancer growth", CELLULAR IMMUNOLOGY, ACADEMIC PRESS, SAN DIEGO, CA, US, vol. 360, 18 December 2020 (2020-12-18), XP086468304, ISSN: 0008-8749, DOI: 10.1016/J.CELLIMM.2020.104262 [retrieved on 2020-12-18]
- TANG, X.J. et al.: "Therapeutic Potential of CAR-T Cell -Derived Exosomes: a Cell -Free Modality for Targeted Cancer Therapy", Oncotarget, vol. 6, no. 42, 29 December 2015 (2015-12-29), pages 44179-44190, XP055585353, ISSN: 1949-2553
- KOOIJMANS, S.A.A. et al.: "Display of GPI-Anchored Anti-EGFR Nanobodies on Extracellular Vesicles Promotes Tumour Cell Targeting", Journal of Extracellular Vesicles, 16 January 2016 (2016-01-16), page 31053, XP055416987, ISSN: 2001-3078
- DATABASE BIOSIS [Online] BIOSCIENCES INFORMATION SERVICE, PHILADELPHIA, PA, US; 7 December 2017 (2017-12-07), BAO HUIJING ET AL: "Chimeric Antigen Receptor-Engineered Exosome As a Drug Delivery System in Mantle Cell Lymphoma", Database accession no. PREV201800670523 & BLOOD, vol. 130, no. Suppl. 1, 7 December 2017 (2017-12-07), page 5561, 59TH ANNUAL MEETING OF THE AMERICAN-SOCIETY-OF-HEMATOLOGY (ASH); ATLANTA, GA, USA; DECEMBER 09 -12, 2017 ISSN: 0006-4971(print)

## Description

### TECHNICAL FIELD

The present invention relates to the technical field of biomedicine, and more specifically, to a method for preparing chimeric antigen receptor (CAR)-carrying exosomes through isolation, and use of the CAR-carrying exosomes in treatment of diseases.

### BACKGROUND

Current strategies for treating malignant tumors primarily include surgical therapy, radiotherapy, and chemotherapy. New targeted therapies are generally thought of as auxiliary. These hierarchical practices have produced positive results. However, the recurrence, metastasis and therapeutic tolerance of malignant tumors are still problems that have always plagued clinical and scientific researchers. In recent years, methods for genetically-modifying various immune cells to treat diseases have been proposed. The expression of chimeric antigen receptor (CAR) on T cells, for example, is achieved so that genetically-modified T cells can target antigens expressed on tumor cells to treat cancer. This type of treatment has achieved a certain degree of success, and the first like product was also approved by the Food and Drug Administration in 2017. (Brentjens R, et al. Treatment of chronic lymphocytic leukemia with genetically targeted autologous T cells: case report of an unforeseen adverse event in a phase I clinical trial, Molecular Therapy, 2010, 18 (4): 666-668.).

With the development of current technologies, the CAR constructed by CAR cell technology at present mainly includes the following three generations. The first-generation CAR consists of extracellular hinge region (single-chain fragment variable (scFv)), transmembrane region (TM) and intracellular signaling region (immunoreceptor tyrosine-based activation motif (ITAM)), and the parts of CAR are linked as follows: scFv-TM-CD3ζ (Zhang T, Barber A, Sentman C L., Chimeric NKG2D-Modified T Cells Inhibit Systemic T-Cell Lymphoma Growth in a Manner Involving Multiple Cytokines and Cytotoxic Pathways[J]. Cancer research, 2007, 67 (22): 11029-11036.).

The second-generation CAR is developed subsequently by adding the intracellular signaling region of CD28 or CD137 (also known as 4-1BB) on the basis of the first generation, and the parts of CAR are linked as follows: scFv-TM-CD28-ITAM or scFv-TM-CD137-ITAM. The costimulation of B7/CD28 or 4-1BBL/CD137 in the intracellular signaling region causes the continuous proliferation of T cells or other immune cells, and can promote the secretion of IL-2 and other cytokines by T cells (Savoldo B, et al., CD28 costimulation improves expansion and persistence of chimeric antigen receptor-modified T cells in lymphoma patients, The Journal of Clinical Investigation, 2011, 121 (5): 1822.).

The third-generation CAR developed in recent years has parts linked as follows: scFv-TM-CD28-CD137-ITAM or scFv-TM-28-CD134-ITAM, which can further improve the survival cycle and effect of CAR-T in the body (Carpenito C, et al., Control of Large, Established Tumor Xenografts With Genetically Retargeted Human T Cells Containing CD28 and CD137 Domains, Proceedings of the National Academy of Sciences, 2009, 106 (9): 3360-3365.). In recent years, in addition to the most commonly used T cells, other types of immune cells have also been used for the treatment by the CAR technology, such as CAR-NK (Chu J, et al., CS1-specific chimeric antigen receptor (CAR)-engineered natural killer cells enhance in vitro and in vivo antitumor activity against human multiple myeloma. Leukemia, 2014, 28 (4): 917-927.).

CAR cells have bright prospects in clinical applications such as tumor immunotherapy. However, there are currently the following obvious problems: In the case where the autologous immune cells are used:
(1) It takes 10 to 14 days to achieve the retransfusion of cells for a patient after blood collection, which results in the miss of the perfect time for treating the patient.
(2) A patient often undergoes multiple treatments such as radiotherapy and chemotherapy, so the patient have poor physical conditions and immune cells with low activity, resulting in that the effectiveness of the retransfused cells cannot be guaranteed.
(3) It may be inappropriate to collect blood from a patient with an advanced malignant disease.
(4) The massive retransfusion of immune cells and the substantial proliferation of immune cells may cause inflammatory storm and thus lead to a dangerous complication of clinical treatment.
(5) The use of donor-derived CAR expressing immune cells is likely to cause immune rejection.

It should be noted that immune cells can secrete a large number of exosomes, which have a diameter of 30 nm to 150 nm and a density of 1.13 g/mL to 1.19 g/mL. The exosomes express specific proteins that carry important signaling molecules of immune cells, including proteins, lipids, RNA and the like, and retain the similar biological activities to parental immune cells. When immune cells are activated, exosomes have some potential to kill cells.

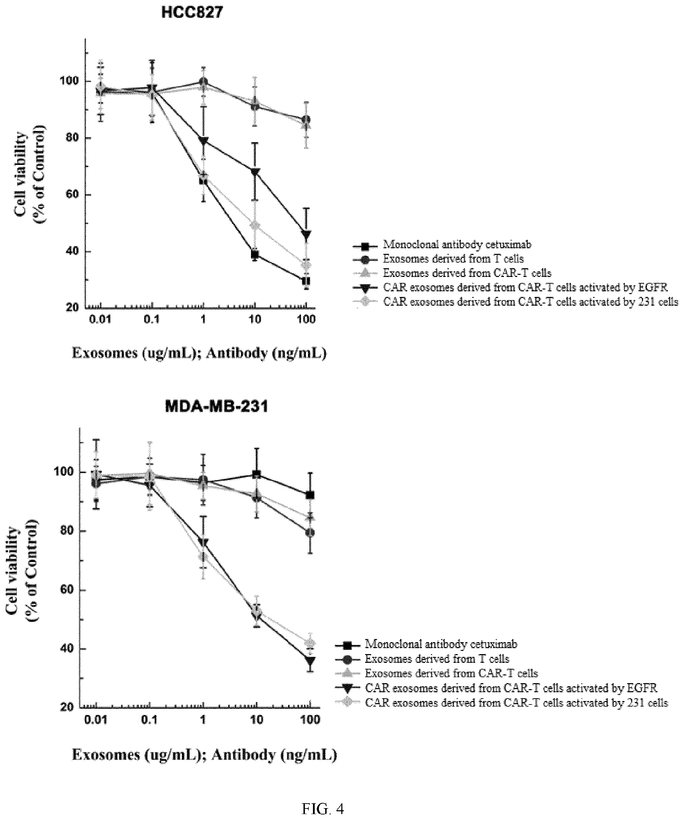

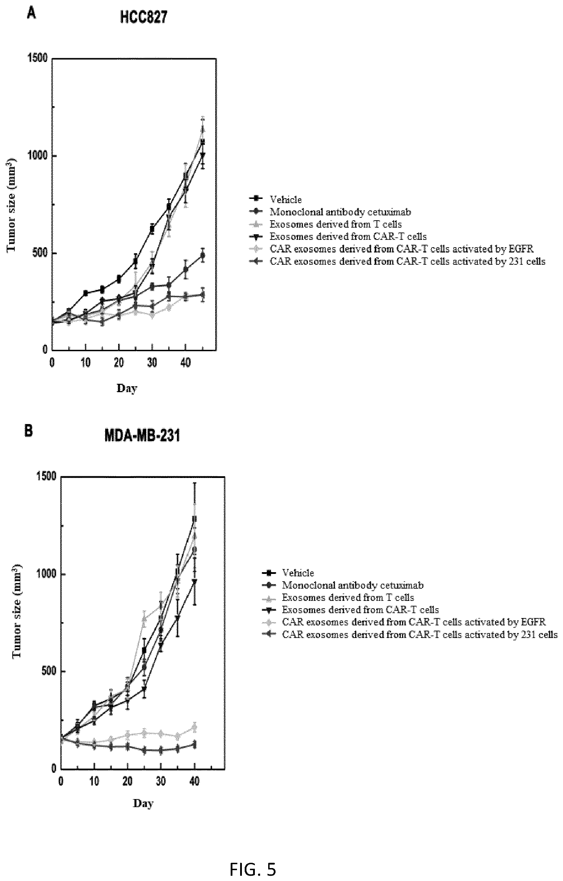

In non-patent literature review (Tang X J, et al. Therapeutic potential of CAR-T cell-derived exosomes: a cell-free modality for targeted cancer therapy. Oncotarget, 2015, 6 (42): 44179.), the possibility of using exosomes secreted by CAR-T cells to treat cancer is proposed. However, the follow-up studies have shown that the exosomes secreted by CAR-T cells have a complicated composition, and exhibit no specific targetability and no real tumor-killing effect. No significant tumor suppression effect is observed during an *in-vivo* experiment where directly-isolated exosomes of CAR-T cells are used to treat tumors (FIG. 4 and FIG. 5). So far, there is no report that exosomes derived from CAR expressing immune cells are effective in treating diseases. The new method for cell-free treatment of diseases using exosomes secreted by CAR-T cells is facing serious challenges.

Recently, the inventors have conducted in-depth research on the composition of exosomes secreted by CAR immune cells, and the results show that, although exosomes of CAR immune cells have a poor effect in tumor treatment, specific exosomes carrying CAR proteins that exhibit a very strong anti-tumor effect. The specific exosomes carrying CAR proteins are prepared as follows: specific antigens are used to stimulate CAR-T cells so that CAR-T cells are active to specific antigens, and then the secreted exosomes are purified and enriched to obtain specific exosomes carrying CAR proteins. The exosomes can be further engineered due to their membranous structure, such as coated with toxins or coated with radioactive particles, so as to realize the treatment of tumors and other diseases.

Information disclosed in this background section is provided merely for increasing the comprehension of the general background of the present invention, and shall not be regarded as acknowledgement or any form of suggestion that the information constitutes the prior art commonly known to those of ordinary skill in the art.

### SUMMARY

The present invention is intended to provide a method for preparing CAR-carrying exosomes (hereinafter referred to as "CAR exosomes") derived from CAR expressing immune cells, and use of the CAR-carrying exosomes in treatment of diseases.

In a first aspect of the present invention, a method for preparing CAR exosomes is provided, including the following step:
A) Preparation of CAR expressing immune cells

It should be noted that the CAR expressing immune cells in this step can be prepared by any of methods mentioned in many references, such as Johnson L A, et al. Rational development and characterization of humanized anti-EGFR variant III chimeric antigen receptor T cells for glioblastoma, Science Translational Medicine, 2015, 7 (275): 275ra22-275ra22; Park S, et al. Micromolar affinity CAR T cells to ICAM-1 achieves rapid tumor elimination while avoiding systemic toxicity, Scientific Reports, 2017, 7 (1): 14366.; Li N, et al. Therapeutically targeting glypican-2 via single-domain antibody-based chimeric antigen receptors and immunotoxins in neuroblastoma, Proceedings of the National Academy of Sciences, 2017, 114 (32): E6623-E6631; Chu J, et al. CS1-specific chimeric antigen receptor (CAR)-engineered natural killer cells enhance in vitro and in vivo antitumor activity against human multiple myeloma, Leukemia, 2014, 28 (4): 917-927; and others. There is no essential difference between the preparation method adopted in the present invention and methods discussed in the above-mentioned references, and CAR expressing immune cells prepared by a method reported in the above-mentioned references or a general bioengineering technology can be used in the present invention. The immune cells are T cells, NK cells, and the like. The immune cells can be derived from a patient or a healthy donor.

In a specific embodiment of the present invention, the immune cells are T cells derived from a healthy donor, and CAR expressing immune cells are prepared according to the following steps:
(1) activating an isolated cell sample where, the sample includes T cells or T cell progenitors;
(2) constructing a viral vector carrying scFv-CD8 hinge and TM-4-1BB-CD3;
(3) constructing a recombinant plasmid to package a virus;
(4) infecting T cells with the virus; and
(5) expanding obtained CAR-T cells *in vitro.*

In another specific embodiment of the present invention, the immune cells are NK cells. A viral vector is constructed for scFv-hinge-TM-CD28-CD3, a recombinant plasmid is constructed to package a virus, and then NK cells are infected with the virus. CAR-NK cells are expanded *in vitro.* In another specific embodiment of the present invention, the immune cells are CAR-T cells, and a viral vector carrying scFv-hinge-CD28-4-1BB-CD3 is constructed.

### B) Antigen-specific activation of CAR expressing immune cells

After a large number of CAR expressing immune cells are obtained, the CAR expressing immune cells require antigen-specific activation.

The activating agents used in this step is a soluble recombinant antigen-protein, engineered cells expressing a specific target, tumor cells expressing a specific target, or the like. The specific target refers to an antigen recognized by the scFv expressed in the CAR expressing immune cells, namely, a specific antigen targeted by the CAR expressing immune cells. It should be noted that, compared with a soluble recombinant protein, an immobilized soluble recombinant protein can achieve a better activation effect, such as magnetic beads coated with recombinant antigens. It should also be noted that activating agents derived from living cells often need to be inactivated.

The antigen targeted by scFv in CAR expressing immune cells can be EGFR, HER2, CD20 and other targets commonly used in targeted therapy at present (Caruso H G, et al., Tuning sensitivity of CAR to EGFR density limits recognition of normal tissue while maintaining potent antitumor activity[J]. Cancer Research, 2015, 75(17): 3505-3518; and Ahmed N, et al. Human Epidermal Growth Factor Receptor 2 (HER2)-Specific Chimeric Antigen Receptor-Modified T Cells for the Immunotherapy of HER2-Positive Sarcoma. Journal of Clinical Oncology, 2015, 33 (15): 1688-1696), and can also be CD19, Mesothelin and other tumor antigens (Turtle C J, et al. CD19 CAR-T cells of defined CD4+: CD8+ composition in adult B cell ALL patients, The Journal of clinical investigation, 2016, 126 (6): 2123.). In principle, CAR expressing immune cells recognizing any target can be used in this step.

In a specific embodiment of the present invention, the CAR expressing immune cells used are CAR-T cells. The scFv of the CAR-T cells targets to EGFR.

In another specific embodiment of the present invention, the CAR expressing immune cells used are CAR-NK cells. The scFv of the CAR-NK cells targets to HER2.

The activation is conducted as follows: adding antigen protein or immobilized antigen protein to an *in vitro* culture system, directly co-cultivating CAR expressing immune cells with inactivated engineered cells expressing specific target, directly co-cultivating CAR expressing immune cells with inactivated tumor cells expressing specific target, or the like.

The activating agent can specifically be: epidermal growth factor receptor (EGFR) extracellular domain recombinant protein, EGFR extracellular domain recombinant protein cross-linked with magnetic beads, CHO cells expressing EGFR, or MDA-MB-231 cells expressing EGFR; or HER2 extracellular domain recombinant protein cross-linked with magnetic beads, BT474 cells expressing HER2, or the like.

In a specific embodiment of the present invention, the activating agent is EGFR extracellular domain recombinant protein coupled with magnetic beads, or inactivated MDA-MB-231 cells highly-expressing EGFR. In this embodiment, the activation is conducted specifically as follows: cultivating CAR-T cells in a medium with the EGFR extracellular domain recombinant protein coupled with magnetic beads, or co-cultivating CAR-T cells with the inactivated MDA-MB-231 cells highly-expressing EGFR.

In another specific embodiment of the present invention, the activating agent is HER2 extracellular domain recombinant protein coupled with magnetic beads or BT474 cells highly-expressing HER2. The activation is conducted specifically as follows: cultivating CAR-NK cells in a medium with the HER2 extracellular domain recombinant protein coupled with magnetic beads, or co-cultivating CAR-NK cells with the inactivated BT474 cells highly-expressing HER2.

It should be noted that it is basically impossible to obtain CAR exosomes if the step B is directly skipped. That is, the exosomes directly obtained from isolation and purification without specific antigen activation of CAR expressing immune cells include very low content of CAR exosomes, and basically cannot be used for disease treatment or scientific research. However, it does not rule out that large-scale cultivation can be conducted for enrichment and purification, but there is no practical value with respect to economic considerations.

### C) Isolation of exosomes secreted by CAR expressing immune cells

The culture supernatant is collected depending on the activation method. The exosomes are isolated by a general exosome isolation method. The exosomes can be isolated by any of methods mentioned in many references, such as Théry C, et al., Isolation and characterization of exosomes from cell culture supernatants and biological fluids, Current Protocols in Cell Biology, 2006: 3.22. 1-3.22. 29; Coumans F A W, et al., Methodological Guidelines to Study Extracellular Vesicles, Circulation research, 2017, 120 (10): 1632-1648; Li L, et al., Human bile contains MicroRNA-laden extracellular vesicles that can be used for cholangiocarcinoma diagnosis, Hepatology, 2014, 60 (3): 896-907; and Li L, Piontek K, Ishida M, et al., Extracellular vesicles carry microRNA-195 to intrahepatic cholangiocarcinoma and improve survival in a rat model, Hepatology, 2017, 65 (2): 501-514. There is no essential difference between the technical means for isolating exosomes in this step and the methods mentioned in above references.

In a specific embodiment of the present invention, the exosomes are isolated as follows: centrifuging the collected culture supernatant at 4°C and 2,000 g for 10 min to remove dead cells and large debris; carefully transferring the resulting supernatant to a new sterile centrifuge tube, and then centrifuging at 4°C and 10,000 g for 30 min to remove organelles and small particles; carefully transferring the resulting supernatant to a sterile ultracentrifuge tube, and ultracentrifuging at 4°C and 110,000 g for 70 min; carefully discarding the supernatant, and washing the precipitates with PBS once; and ultracentrifuging a resulting suspension at 4°C and 110,000 g for 70 min to obtain precipitates, namely, exosomes.

### D) Purification and Enrichment of CAR exosomes

In this step, the CAR exosomes are purified and enriched base on the specific binding affinity of CAR to an antigen. In order to increase purity, in some specific application embodiments, the exosomes secreted by immune cells can be initially purified using the binding of protein L to the immunoglobulin light chain. It should be noted that this method cannot replace the step of purifying using an antigen.

The steps are as follows:
Incubating magnetic beads coated with a specific antigen (namely, CAR-capturing magnetic beads) with the exosome suspension obtained in step C, where, the magnetic beads include a recombinant target protein antigen that can specifically bind to CAR; after incubation, placing the suspension in a magnetic field; removing the supernatant, and then adding washing buffer; placing the resulting suspension to a sorting column, eluting the exosomes retained on the column with elute buffer, where, after the suspension_is placed in the column, substances flowing out first are exosomes without the antigen binding ability, and then the column is rinsed with elute buffer to obtain the CAR-carrying exosomes with the antigen binding ability; based on the volume of the initially exosome suspension used, adding PBS as appropriate to resuspend CAR exosomes; detecting the total protein concentration with a Bradford kit; and dispensing and storing the resulting exosomes at -80°C.

In a specific embodiment of the present invention, magnetic beads coated with EGFR recombinant protein are used, and the magnetic beads are Dynabeads. The magnetic beads are added to a suspension with the exosomes. The test tube with the suspension is placed in a magnetic field, and the exosomes specifically binding to the magnetic beads are fixed in the magnetic field. After the magnetic beads are fixed, the supernatant is removed and the test tube is taken out from the magnetic field. The resulting exosomes are resuspended with PBS and then added to a column. The unbound components flowing out first are collected, and the column is rinsed with buffer to obtain the exosomes without the antigen binding ability. The column is taken out from the magnetic field, and the exosomes retained on the column are quickly eluted out with buffer and balanced to physiological pH, which are the CAR exosomes.

In another specific embodiment of the present invention, in this step, the mixture of the magnetic beads coated with protein L and the PBS solution with exosomes is first incubated at 4°C for 60 min. The magnetic beads bind to the corresponding exosomes through the specific binding of protein L to the immunoglobulin light chain. The test tube with the mixture is placed in a magnetic field, and the exosomes binding to the magnetic beads are fixed in the magnetic field. After the magnetic beads are fixed, the supernatant is removed and the test tube is taken out from the magnetic field. The resulting exosomes are resuspended with PBS and then added to a column. The unbound components flowing out first are collected, and the column is rinsed with buffer to obtain the exosomes without immunoglobulins. The column is taken out from the magnetic field, and the exosomes retained on the column are quickly eluted out with buffer. Immediately after the pH is restored, the obtained exosomes are incubated with recombinant HER2 protein-coated magnetic beads at 4°C for 30 min, and then the suspension is placed in a magnetic field. The exosomes binding to the magnetic beads are fixed once again by the magnetic field. After the magnetic beads are adsorbed, the supernatant is removed and the test tube is taken out from the magnetic field. The resulting exosomes are resuspended with PBS and then added to a column. The unbound components flowing out first are collected, and the column is rinsed with buffer to obtain the exosomes without CAR. The column is taken out from the magnetic field, and the CAR-carrying exosomes retained on the column are quickly eluted out with buffer and balanced to physiological pH, which are the target exosomes.

In a second aspect of the present invention, a CAR exosome derived from antigen-specific activated immune cells wherein the immune cells are T cells or NK cells activated with antigen prepared by the preparation method described above is provided.

In the present invention, the biological activity assays are conducted for the above CAR exosomes. The exosomes carry CAR proteins, and have an average diameter of about 30 nm to 150 nm, and a morphology observed under the transmission electron microscope (TEM) consistent with the characteristics of exosomes. Further studies have shown that the CAR-carrying exosomes derived from immune cells can well target to cells and tissues expressing the target, and can inhibit the proliferation of tumor cells and the growth *of in vivo* tumors.

In a third aspect of the present invention, use of the aforementioned CAR exosomes and a composition thereof in preparation of anti-tumor drugs is provided.

The tumor mentioned in the present invention includes adenocarcinoma, leukemia, lymphoma, melanoma, and sarcoma. The source of tumor tissue includes, but is not limited to, adrenal gland, gallbladder, bone, bone marrow, brain, breast, bile duct, gastrointestinal tract, heart, kidney, liver, lung, muscle, ovary, pancreas, parathyroid gland, penis, prostate, skin, salivary gland, spleen, testis, thymus, thyroid and uterus. In addition to the above-mentioned tumors, the present invention can also be used for central nervous system tumors such as glioma and astrocytoma; ocular tumors including basal cell carcinoma, squamous cell carcinoma, melanoma, and the like; endocrine tumors such as neuroendocrine system tumors and gastro-entero-pancreatic endocrine system tumors; reproductive system tumors; head and neck tumors; and the like; which are not listed in detail here.

Further, the tumor is non-small-cell lung carcinoma (NSCLC) or breast cancer. In a specific embodiment of the present invention, the CAR exosomes inhibit the cell viability and tumor growth rate of MDA-MB-231 and HCC827, especially of MDA-MB-231 that is naturally resistant to cetuximab. In another specific embodiment of the present invention, the CAR exosomes inhibit the cell viability of BT474.

Further, the CAR exosomes are membranous nanovesicles, which can be further modified by a liposome-related engineering method, or coated with chemotherapeutic drugs, radioactive ions, or the like.

In a specific embodiment, CAR exosomes are further engineered to be loaded with adriamycin and exhibit cytotoxic effects on breast cancer cells.

The anti-tumor drugs mentioned in the present invention refer to drugs capable of inhibiting and/or treating tumors, which may include delaying the development of symptoms associated with tumor growth and/or reducing the severity of these symptoms, alleviating existing symptoms associated with tumor growth and preventing the occurrence of other symptoms, and reducing or preventing the metastasis.

Further, the CAR exosomes are used in combination with anti-tumor drugs.

The CAR exosomes and a composition thereof disclosed in the present invention can also be used for treating tumors, in combination with other anti-tumor drugs or radiotherapy. These antitumor drugs or radiotherapies include:
1. cytotoxic drugs: (1) drugs that act on the chemical structure of DNA: alkylating agents such as nitrogen mustards, nitrosourea and methanesulfonate; platinum compounds such as cisplatin, carboplatin, and oxaliplatin; and mitomycin (MMC); (2) drugs that affect the synthesis of nucleic acids: dihydrofolate reductase (DHFR) inhibitors such as methotrexate (MTX) and Alimta; thymidine synthase inhibitors such as fluorouracil (5FU, FT-207, and capecitabine); purine nucleoside synthase inhibitors such as 6-mercaptopurine (6-MP) and 6-TG; nucleotide reductase inhibitors such as hydroxyurea (HU); DNA polymerase inhibitors such as cytarabine (Ara-C) and Gemzar (Gemz); (3) drugs that act on nucleic acid transcription: drugs that selectively act on DNA templates to inhibit DNA-dependent RNA polymerase and thus inhibit the synthesis of RNA, such as actinomycin D, daunorubicin, adriamycin, epirubicin, aclarubicin, and mithramycin; (4) drugs that mainly act on the synthesis of tubulin: paclitaxel, taxotere, vinblastine (VBL), vinorelbine (NVB), podophyllotoxin (PPT), and homoharringtonine; (5) other cytotoxic drugs: asparaginase which mainly inhibits the synthesis of protein;
2. hormones: anti-estrogens: tamoxifen, droloxifene, exemestane, and the like; aromatase inhibitors: aminoglutethimide, formestane, letrozole, arimidex, and the like; and anti-androgens: flutamide RH-LH agonists/antagonists: goserelin, enatone, and the like;
3. biological response modifiers (BRMs) which mainly inhibit tumors through immune functions in the body: interferon, interleukin-2; thymosin;
4. monoclonal antibodies: Rituximab (MabThera), Herceptin (Trastuzumab), and Bevacizumab (Avastin);
5. various radiotherapies; and
6. some drugs that have unclear mechanisms at present and need to be further studied: cell differentiation inducers such as retinoids; and apoptosis inducers. The CAR-carrying exosomes derived from immune cells and a composition thereof disclosed in the present invention can be used in combination with one or a combination of the aforementioned anti-tumor drugs.

In a fourth aspect of the present invention, use of the above-mentioned CAR exosomes and a composition thereof in preparation of drugs for treating severe infectious diseases or autoimmune diseases is provided.

In a fifth aspect of the present invention, a preparation is provided, which is a composition including the CAR-exosomes described above.

The preparation is a composition including CAR exosomes, which can exhibit a significant anti-tumor effect after being administered to animals including humans by injection or other manners. Specifically, the composition is effective in preventing and/or treating tumors, and can be used as an anti-tumor drug. In addition, due to the nature of immune cells, the exosomes and the composition of exosomes can also be used to fight against other diseases, such as severe infectious diseases and autoimmune diseases.

In a sixth aspect of the present invention, a method for prolonging the recurrence-free survival of a cancer patient who is ready to receive, is receiving or has received cancer treatment (such as chemotherapy, radiotherapy, targeted therapy and/or surgery) by administering a therapeutically effective amount of CAR exosomes to the patient is provided. Compared with CAR immune cell therapy, exosomes should be more advantageous in the treatment of solid tumors due to the tissue infiltrating ability thereof.

In the present invention, when the CAR exosomes and the composition thereof are administered to animals including humans, the dosage varies with the age and body weight of the patient, the characteristics and severity of the disease, and the route of administration. The total dosage can be defined within a certain range with reference to results of animal experiments and various other conditions.

The present invention has the following advantages:
In the present invention, CAR cells (such as CAR-T cells) are activated with specific antigens, and the resulting exosomes are further analyzed, isolated, purified and enriched to finally obtain CAR-carrying exosomes derived from immune cells. The exosomes can be used for treating various diseases, such as cancer and severe infectious diseases. Moreover, the exosomes have the ability to overcome adverse reactions such as inflammatory storm induced by CAR cell immunotherapy, which enhances the tissue infiltrating ability of CAR. The exosomes are easy to be stored and transported, and provide a new strategy for the treatment of related diseases.

### BRIEF DESCRIPTION OF THE DRAWINGS

FIG. 1 shows the antigen-competition ELISA assay for CAR exosomes.
FIG. 2 shows the antibody-competition ELISA assay for CAR exosomes.
FIG. 3 shows the morphology of CAR exosomes derived from CAR-T activated by a recombinant EGFR protein under an electron microscope.
FIG. 4 shows the inhibition of CAR exosomes derived from CAR-T on the growth of MDA-MB-231 and HCC827 cells *in vitro.*
FIG. 5 shows the growth curves of MDA-MB-231 and HCC827 cell tumors under the inhibition of CAR exosomes derived from CAR-T.
FIG. 6 shows the morphology of CAR exosomes derived from CAR-NK activated by a recombinant HER2 protein under an electron microscope.
FIG. 7 shows the inhibition of CAR exosomes derived from CAR-NK and a composition thereof on the growth of BT474 and MCF-7 cells *in vitro.*
FIG. 8 shows the effect of CAR exosomes derived from CAR-T on the apoptosis of cells.

### DETAILED DESCRIPTION OF THE EMBODIMENTS

The specific implementations provided in the present invention will be further described in detail below with reference to examples.

The following examples and experimental examples are provided to further illustrate the present invention, and shall not be construed as a limitation to the present invention. The examples do not include detailed descriptions of traditional methods, such as methods for constructing vectors and plasmids, methods for inserting genes encoding proteins into such vectors and plasmids, methods for introducing plasmids into host cells, methods for packaging viruses, and methods for infecting immune cells with viruses to achieve the expression of target proteins. Such methods are well known to those of ordinary skill in the art, and are described in many publications, including Sambrook, J., Fritsch, E. F. and Maniais, T. (1989) Molecular Cloning: A Laboratory Manual, 2nd edition, Cold spring Harbor Laboratory Press; Buchschacher, G. L., Jr., and Wong-Staal, F. (2000) Development of Lentiviral Vectors for Gene Therapy for Human Diseases. Blood 95, 2499-2504; Yee, J.-K., Miyanohara, A., LaPorte, P., Bouic, K., Burns, J. C., and Friedmann, T. (1994) A General Method for the Generation of High-Titer, Pantropic Retroviral Vectors: Highly Efficient Infection of Primary Hepatocytes. Proc. Natl. Acad. Sci. USA 91, 9564-9568; Yee, J. K. (1999) in The Development of Human Gene Therapy (Friedmann, T., ed), pp. 21-45, Cold Spring Harbor Laboratory Press, Cold Spring Harbor, NY; Yee, J. K., Moores, J. C., Jolly, D. J., Wolff, J. A., Respess, J. G., and Friedmann, T. (1987) Gene Expression from Transcriptionally Disabled Retroviral Vectors. Proc. Natl. Acad. Sci. USA 84, 5197-5201; and others.

### Example 1: Preparation of CAR exosomes derived from CAR-T cells

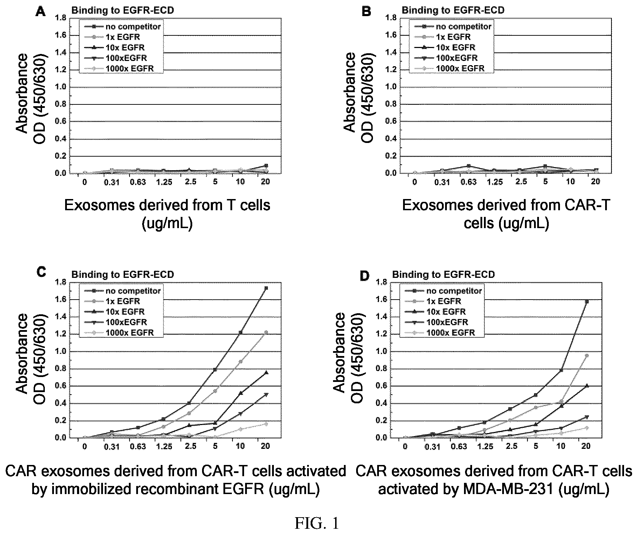

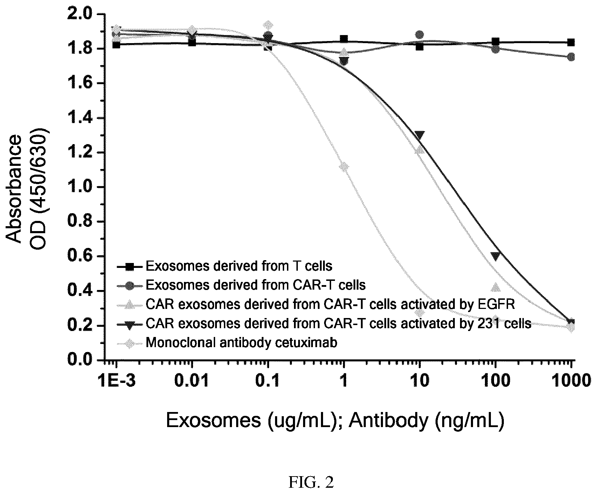

1) Gene synthesis of a CAR sequence with anti-EGFR scFv (the gene synthesis was entrusted to GENEWIZ): The scFv sequence was derived from the anti-EGFR antibody cetuximab (Li et al., 2005, Structural basis for inhibition of the epidermal growth factor receptor by cetuximab, Cancer Cell, 7: 301-311), and the specific structure of CAR included: anti-EGFR scFv-CD8α hinge region and transmembrane region-4-1BB co-activation domain, and a CD3ζ signaling molecule intracellular domain. The specific sequence was roughly the same as that reported in Johnson L A, et al. Science translational medicine, 2015, 7 (275) (except scFv). For ease of detection, a Myc tag was inserted between the scFv and the hinge region at the same location as described in Chu J, et al. CS1-specific chimeric antigen receptor (CAR)-engineered natural killer cells enhance in vitro and in vivo antitumor activity against human multiple myeloma. Leukemia, 2014, 28 (4): 917-927. The entire sequence was cloned into the pLenti6.3/v5 lentiviral vector (Invitrogen) with a CMV promoter by homologous recombination.
2) Preparation of lentiviruses using HEK293T cells: the preparation of lentiviruses is well known to those of ordinary skill in the art, and thus will not be detailed here. The brief steps were as follows: a suitable amount of HEK-293T cells were co-transfected with the constructed lentiviral vectors and the viral packaging plasmids (Mission viral packaging plasmids, Sigma-Aldrich); and then 72 h after the transfection, the viruses were collected and purified by concentration (Lenti-X concentrator, Clontech).
3) Isolation and cultivation of T cells and preparation of CAR-T: Fresh peripheral blood mononuclear cells (PBMCs) were isolated by density gradient centrifugation, and then stimulated and enriched using paramagnetic beads coupled with anti-CD3 and anti-CD28 antibodies (Dynabeads ClinExVivo CD3/CD28, Invitrogen, Camarillo, CA, USA). The paramagnetic beads and cells were used at a ratio of (2-3):1. The cells were diluted to a concentration of 5 × 10 ⁶/mL to 8 × 10 ⁶/mL, and incubated in a medium supplemented with IL-2 for 24 h. The obtained T cells were infected with lentiviruses repeatedly. The cells were counted and the medium was replaced every other day. When the T cells exhibited a resting state, the subsequent experiment was conducted. The so-called resting state means that the cell counts show a decreased proliferation coefficient and the cell size stops changing. The cell supernatants before and after transfection were collected to extract exosomes for comparison. The extraction method will be described later.
4) Antigen-specific activation of T cells: In this step, two methods were used to achieve the antigen-specific activation of CAR-T cells. One method was: adding EGFR extracellular domain recombinant protein coupled with magnetic beads to a T cell culture medium. The protein concentration was between 5 ug/mL and 1 mg/mL, and a concentration gradient was adopted for the experiment. The culture supernatant was collected 24 h after the cultivation. The recombinant protein in the supernatant was removed by a magnetic field. The other method was: co-cultivating the CAR-T cells and the tumor cells MDA-MB-231 with high expression of EGFR. Before cultivation, MDA-MB-231 cells were inactivated with 100 Gy of gamma rays. The CAR-T cells and the tumor cells were co-cultivated at a ratio of 2:1, 4:1 and 8:1 separately, and the culture supernatant was collected 24 h after the co-cultivation.
5) Isolation of exosomes: The exosomes were extracted from the culture supernatants described in steps 3) and 4) according to the following steps: the culture supernatant was put in a 500 mL sterile centrifuge bottle or a 50 mL polypropylene centrifuge tube (purchased from Beckman) and then centrifuged at 4°C and 2,000 g for 10 min to remove dead cells and large debris; the resulting supernatant was carefully transferred to a new sterile centrifuge tube and then centrifuged at 4°C and 10,000 g for 30 min to remove organelles and small particles; the resulting supernatant was carefully transferred to a sterile ultracentrifuge tube and then ultracentrifuged at 4°C and 110,000 g (Beckman ultracentrifuge) for 70 min; the resulting supernatant was carefully discarded, and the precipitates were washed once with saline injection; and the resulting suspension was ultracentrifuged at 4°C and 110,000 g for 70 min to obtain precipitates, namely, exosomes. Depending on a volume of an initially collected culture supernatant, PBS was added as appropriate to resuspend the exosomes.
6) Preparation of CAR-carrying exosomes:
   The CAR-carrying exosomes were purified and enriched from the obtained exosomes according to the following steps: Magnetic beads coated with an EGFR extracellular domain recombinant protein were added to a saline solution with the exosomes, and the resulting mixture was incubated at 4°C for 30 min. The magnetic beads bound to CAR exosomes with the corresponding scFv through specific antigen-antibody interaction. The test tube with the mixture was placed in a magnetic field, and the exosomes binding to the magnetic beads were fixed in the magnetic field. After the magnetic beads were fixed, the supernatant was removed and the test tube was taken out from the magnetic field. The resulting exosomes were resuspended with PBS and then added to a column. The unbound components flowing out first were collected, and the column was rinsed with buffer to obtain the exosomes without CAR. The column was then taken out from the magnetic field, and the CAR-carrying exosomes retained on the column were quickly eluted out with buffer, which were the target exosomes. After a total protein concentration was detected with a Bradford kit (purchased from Thermo), exosomes could be dispersed and stored at -80°C for a long term.
   It should be noted that CAR exosomes available for subsequent implementation cannot be obtained by purification and enrichment from exosomes secreted by CAR-T cells without antigen-specific activation. That is, the exosomes secreted by CAR-T cells without antigen-specific activation have an extremely-low content of CAR-carrying exosomes.
7) Assay of CAR-carrying exosomes:
   Since the ELISA experiment is a common experiment well known to those of ordinary skill in the art, the experimental methods for which the specific conditions are not noted in the following examples can be conventional methods in the art. For example, the experiment can be conducted with reference to "Molecular Cloning: A Laboratory Manual" (Third edition, New York, Cold Spring Harbor Laboratory Press, 1989) or according to the steps recommended by the supplier of a kit. The CAR expression was determined for exosomes according to the following steps: CAR exosomes were diluted at a certain dilution ratio and then added to a blocked 96-well plate coated with EGFR antigen; the resulting mixture was incubated at 37°C for 1 h and then washed; EGFR recombinant protein was added in a competitive manner, and the resulting mixture was incubated and then washed 3 times with TBST; then the HRP-labeled Anti-Myc antibody was added, and the resulting mixture was incubated at 37°C for 1 h and then washed with TBST; a chromogenic substrate was added, and ELISA assay was conducted; and then calculation and analysis were conducted. The results are shown in FIG. 1. The competitive ELISA with cetuximab was conducted as follows: CAR exosomes were diluted at a certain dilution ratio, and then added, together with biotin-labeled cetuximab, to a blocked 96-well plate coated with EGFR antigen; the resulting mixture was incubated at 37°C for 1 h and then washed 3 times with TBST; then the HRP-labeled avidin (Thermo) was added, and the resulting mixture was incubated at 37°C for 1 h and then washed with TBST; a chromogenic substrate was added, and ELISA assay was conducted; and then calculation and analysis were conducted. The results are shown in FIG. 2.

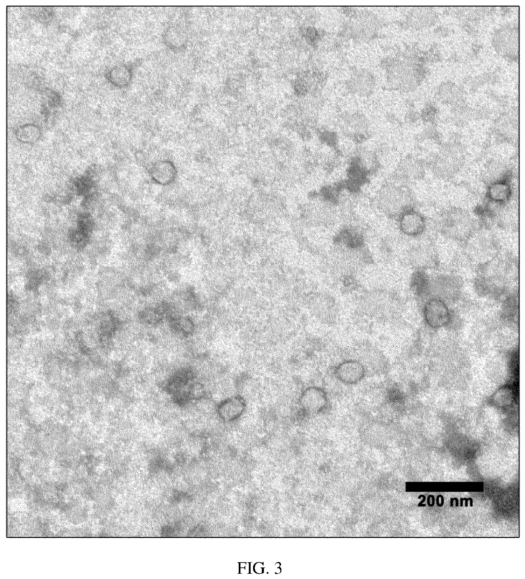

The morphology of the obtained CAR exosomes was observed by TEM: the exosomes were fully resuspended, and then 10 µ1 was pipetted and added dropwise to a sample-supporting copper net, and then kept at room temperature for 5 min; excess liquid was carefully removed with filter paper; uranyl acetate was added dropwise for 2 min of negative staining, excess liquid was removed with filter paper, and the sample was dried under an incandescent lamp; and an image was acquired by TEM at 80 kv to 120 kv. Circular vesicle-like structures with a diameter of about 30 nm to 150 nm were observed. The result is shown in FIG. 3.

The results in this example show that CAR-carrying exosomes derived from immune cells have been successfully obtained, which all express CAR protein, can bind to a specific antigen, and have an average diameter of about 80 nm and a morphology observed under TEM that is consistent with characteristics of exosomes.

### Example 2: Inhibition of CAR exosomes on the viability of EGFR-positive MDA-MB-231 and HCC827 cells

MDA-MB-231 and HCC827 cells (ATCC) at well growth state were taken and diluted to a concentration of 5 × 10³/ml, then inoculated in a 96-well cell culture plate at 200 µl/well, and cultivated in an incubator at 37°C and 5% CO₂ for 24 h. Then EGF with a final concentration of 5 nmol and exosomes with a concentration gradient were added to the culture, and the cetuximab antibody (purchased from Merck) was adopted as a control. Four days later, the cell viability was determined with CellTiter-Glo Luminescent Cell Viability Assay kit (Promega, Madison, WI). The experimental results are shown in FIG. 4. Experimental results show that CAR exosomes can significantly inhibit the viability of MDA-MB-231 and HCC827 cells (P < 0.01, Tukey test), especially of MDA-MB-231 cells that are naturally resistant to cetuximab (FIG. 4).

### Example 3: Inhibition of CAR exosomes on the growth of tumor in vivo

In order to test the anti-tumor activity of CAR exosomes *in vivo,* HCC827 and MDA-231 cells were first subcutaneously inoculated into BALB/c nude mice (Experimental Animal Center, Chinese Academy of Sciences) at the right flank; after tumors were formed, CAR exosomes (3,500 mg/kg) and the antibody cetuximab (10 mg/kg) were injected via the tail vein once a week until the tumors were oversize; and then the mice were sacrificed. The length and width of the tumor were measured every day to calculate the tumor volume.

The tumor growth curves are shown in FIG. 5. The results show that the tumor growth rate in the activated CAR exosome treatment group is significantly lower than that in the cetuximab treatment group (40 days later, P < 0.01, Bonferroni test).

### Example 4: Preparation of CAR exosomes targeting HER2 and derived from CAR-NK cells

1) Gene synthesis of a CAR sequence with anti-HER2 scFv (the gene synthesis was entrusted to GENEWIZ): The scFv sequence was derived from the anti-HER2 antibody trastuzumab (Cho H S, et al. Structure of the extracellular region of HER2 alone and in complex with the Herceptin Fab. Nature, 2003, 421 (6924): 756-760.), and the specific structure of CAR included: anti-HER2 scFv-CD28 hinge region and transmembrane region CD28, and CD3ζ signaling molecule intracellular domain. The specific sequence was the same as that described in Chu J, et al. CS1-specific chimeric antigen receptor (CAR)-engineered natural killer cells enhance in vitro and in vivo antitumor activity against human multiple myeloma. Leukemia, 2014, 28 (4): 917-927 (except ScFv). The entire sequence was cloned into the PCDH lentiviral vector (System Biosciences) with a CMV promoter by homologous recombination.
2) Preparation of lentiviruses using HEK293T cells: The preparation of lentiviruses is well known to those of ordinary skill in the art, and thus will not be detailed here. The brief steps were as follows: a suitable amount of HEK-293T cells were co-transfected with the constructed lentiviral vectors and the viral packaging plasmids pCMV-VSVG and pCMV-dr9; and then 72 h after the transfection, the viruses were collected and purified by concentration (Lenti-X concentrator, Clontech).
3) Preparation of CAR-NK:
   NK-92 cells were diluted to 1 × 10⁶/mL, then cultivated in a medium supplemented with IL-2 overnight, and then repeatedly and continuously infected with lentiviruses. After the third infection, the cells were cultivated in 1640 medium with 20% FBS, which was supplemented with IL-2 at 150 units/ml. The flow cytometry (BD Biosciences, San Jose, CA, USA) was conducted two times to sort cells expressing green fluorescent protein (GFP). The GFP was encoded by a gene carried on the PCDH vector. In addition, the cell supernatants before and after infection were collected during the experiment to extract exosomes for comparison. The extraction method will be described later.
4) Antigen-specific activation of NK cells: In this step, two methods were used to achieve the antigen-specific activation of NK cells. One method was: adding an HER2 extracellular domain recombinant protein coupled with magnetic beads to an NK cell culture medium. The protein concentration was between 5 ug/mL and 1 mg/mL, and a concentration gradient was adopted for the experiment. The culture supernatant was collected 12 h to 24 h after the cultivation. The other method was: co-cultivating the NK cells and the inactivated BT474 cells highly-expressing HER2. The NK cells and the inactivated BT474 cells were co-cultivated at a ratio of 2:1, 4:1 and 8:1 separately, and the culture supernatant was collected 12 h to 24 h after the co-cultivation.
5) Isolation of exosomes: The exosomes were extracted from the culture supernatants described in steps 3) and 4) according to the following steps: the culture supernatant was put in a 500 mL sterile centrifuge bottle or a 50 mL polypropylene centrifuge tube (purchased from Beckman) and then centrifuged at 4°C and 2,000 g for 10 min to remove dead cells and large debris; the resulting supernatant was carefully transferred to a new sterile centrifuge tube and then centrifuged at 4°C and 10,000 g for 30 min to remove organelles and small particles; the resulting supernatant was carefully transferred to a sterile ultracentrifuge tube and then ultracentrifuged at 4°C and 110,000 g (Beckman ultracentrifuge) for 70 min; the resulting supernatant was carefully discarded, and the precipitates were washed once with saline injection; and a resulting suspension was ultracentrifuged at 4°C and 110,000 g for 70 min to obtain precipitates, namely, exosomes. Depending on a volume of an initially collected culture supernatant, PBS was added as appropriate to resuspend the exosomes.
6) Preparation of CAR-carrying exosomes:
   The CAR-carrying exosomes were purified and enriched from the obtained exosomes according to the following steps: Magnetic beads coated with protein L were added to a saline solution with the exosomes, and the resulting mixture was incubated at 4°C for 60 min. The magnetic beads bound to CAR exosomes or immunoglobulin-containing exosomes through the specific binding of protein L to the immunoglobulin light chain. The test tube with the mixture was placed in a magnetic field, and the exosomes binding to the magnetic beads were fixed in the magnetic field. After the magnetic beads were fixed, the supernatant was removed and the test tube was taken out from the magnetic field. The resulting exosomes were resuspended with PBS and then added to a column. The unbound components flowing out first were collected, and the column was rinsed with buffer to obtain the exosomes without immunoglobulins. The column was taken out from the magnetic field, and the exosomes retained on the column were quickly eluted out with a buffer, and then immediately incubated with magnetic beads coated with a recombinant HER2 protein at 4°C for 30 min. The magnetic beads bound to CAR exosomes with the corresponding scFv through specific antigen-antibody interaction. The test tube with the mixture was placed in a magnetic field, and the exosomes binding to the magnetic beads were fixed in the magnetic field. After the magnetic beads were fixed, the supernatant was removed and the test tube was taken out from the magnetic field. The resulting exosomes were resuspended with PBS and then added to a column. The unbound components flowing out first were collected, and the column was rinsed with buffer to obtain the exosomes without CAR. The column was taken out from the magnetic field, and the CAR-carrying exosomes retained on the column were quickly eluted out with buffer and balanced to physiological pH, which were the target exosomes. After a total protein concentration was detected with Bradford kit (purchased from Thermo), exosomes could be dispersed and stored at -80°C for a long term.
7) Assay of CAR-carrying exosomes:
   The morphology of the obtained CAR exosomes was observed by TEM: the exosomes were fully resuspended, and then 10 µl was pipetted and added dropwise to a sample-supporting copper net, and then stood at room temperature for 5 min; excess liquid was carefully sucked off with filter paper; uranyl acetate was added dropwise for 2 min of negative staining, excess liquid was sucked off with filter paper, and the sample was dried under an incandescent lamp; and an image was acquired by TEM at 80 kv to 120 kv. Circular vesicle-like structures with a diameter of about 30 nm to 150 nm were observed. The result is shown in FIG. 6.

### Example 5: Inhibition of NK-derived CAR exosomes on the viability of breast cancer cells

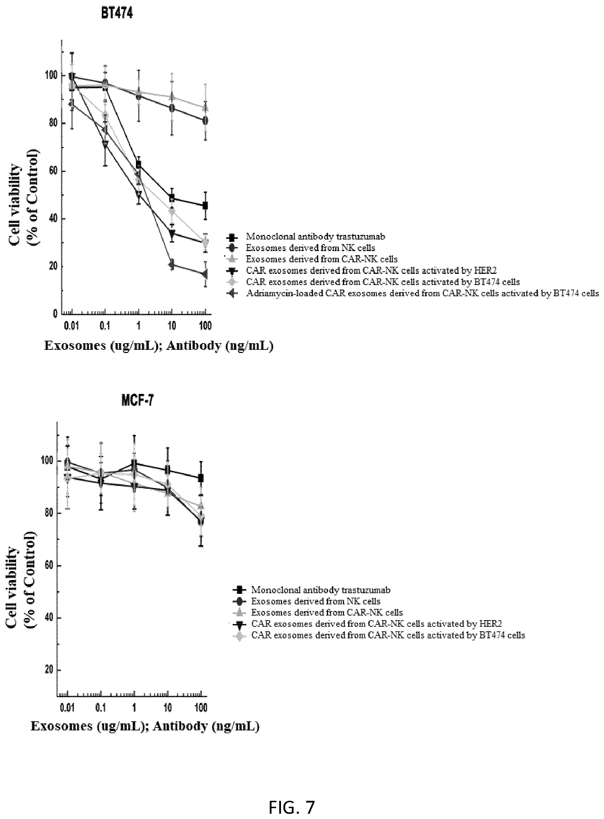

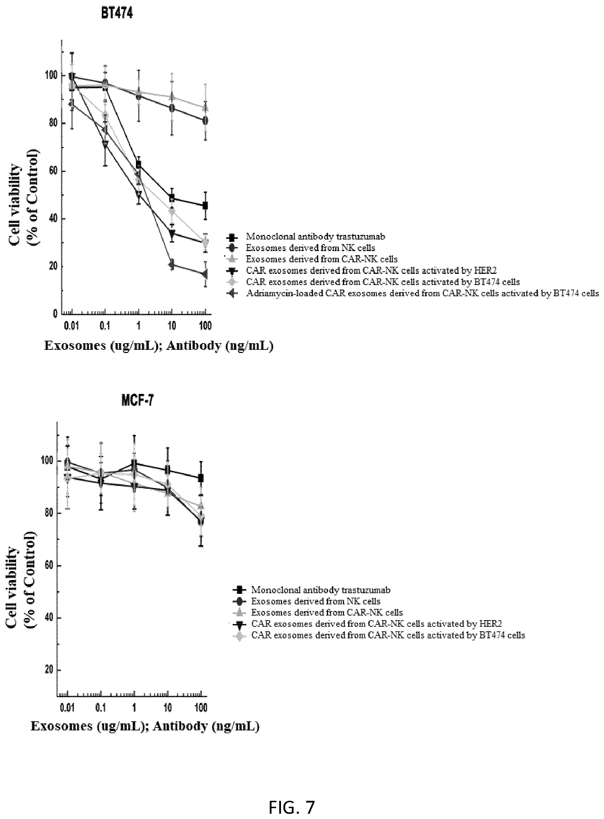

Breast cancer cells BT474 with high expression of HER2 and MCF-7 cells with low expression of HER2 (ATCC) at well growth state were taken and diluted to a concentration of 4 × 10³/ml, then inoculated in a 96-well cell culture plate at 200 µl/well, and cultivated in an incubator at 37°C and 5% CO₂ for 24 h; then exosomes were added to the culture at a concentration gradient, and the trastuzumab antibody was adopted as a control; and 4 days later, the cell viability was determined with CellTiter-Glo Luminescent Cell Viability Assay kit (Promega, Madison, WI). The experimental results are shown in FIG. 7. Experimental results show that CAR exosomes derived from NK cells can significantly inhibit the viability of BT474 cells (P < 0.01, Tukey test), but exhibit a weaker inhibitory effect on MCF-7 cells with low expression of HER2 (FIG. 7).

### Example 6: Preparation of NK-derived CAR exosomes loaded with adriamycin, and the anti-tumor effect of the CAR exosomes

The CAR exosomes obtained in Examples 4 and 5 were mixed with adriamycin at a mass ratio of 1:1, separately. The compound-loaded CAR exosomes were prepared by electroporation. The electroporation was conducted in a 4 mm electroporation cuvette under a voltage of 420 V and a capacitance of 150 µF. Subsequently, free compounds that were not transfected into the exosomes were removed by inverted centrifugation and filtration using ultrafiltration membrane.

It is also possible to introduce the compound into exosomes through lipofection to realize the loading on exosomes. Breast cancer cells BT474 with high expression of HER2 and MCF-7 cells with low expression of HER2 (ATCC) at well growth state were taken and diluted to a concentration of 4 × 10³/ml, then inoculated in a 96-well cell culture plate at 200 µl/well, and cultivated in an incubator at 37°C and 5% CO₂ for 24 h; then exosomes were added to the culture at a concentration gradient; and 4 days later, the cell viability was determined with CellTiter-Glo Luminescent Cell Viability Assay kit (Promega, Madison, WI). The experimental results are shown in FIG. 7. Experimental results show that CAR exosomes loaded with adriamycin can more significantly inhibit the viability of tumor cells (P < 0.01, Tukey test).

### Example 7: Effect of CAR-T-derived CAR exosomes targeting CD20 on the apoptosis of lymphoma cells

1) Gene synthesis of a CAR sequence with the anti-CD20 scFv (the gene synthesis was entrusted to GENEWIZ): The scFv sequence was derived from the anti-CD antibody Rituximab (Du J, et al. Structural basis for recognition of CD20 by therapeutic antibody Rituximab. Journal of Biological Chemistry, 2007, 282 (20): 15073-15080.). The specific structure of CAR included: anti-CD20 scFv-hinge region and CD28 transmembrane region, 4-1BB, and CD3ζ signaling molecule intracellular domain. The specific sequence was the same as that described in Chu J, et al. CS1-specific chimeric antigen receptor (CAR)-engineered natural killer cells enhance in vitro and in vivo antitumor activity against human multiple myeloma. Leukemia, 2014, 28 (4): 917-927 (except ScFv and 4-1BB). The entire sequence was cloned into the PCDH lentiviral vector (System Biosciences) with a CMV promoter by homologous recombination.

The method for preparing lentiviruses using HEK293T cells, the method for preparing CAR-T cells, the method for antigen-specific activation of CAR-T cells, the method for isolating exosomes, and the method for purifying CAR-carrying exosomes are the same as that in the above examples, and thus will not be described here. The activating agent used in the antigen-specific activation of CAR-T cells refers to inactivated Raji cells expressing CD20.

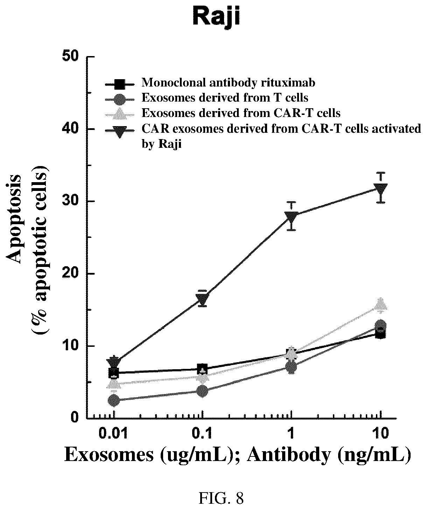

Burkitt lymphoma cells (Raji, ATCC) at a well growth state were taken and diluted to a concentration of 1 × 10⁵/well, and then cultivated in an incubator at 37°C and 5% CO₂ for 24 h; exosomes were added at a concentration gradient to the culture, and the Rituximab antibody was adopted as a control; 16 h after the cultivation, the cells were rinsed and then stained with annexin V-FITC (BD Biosciences); and the flow cytometry was conducted to obtain the apoptosis rate. The experimental results are shown in FIG. 8. Experimental results show that CAR exosomes derived from CAR-T cells can significantly induce the apoptosis of Raji cells and Daudi cells (P < 0.01, Tukey test) (FIG. 8).

The preferred examples of the present invention have been described in detail above, but the present invention is not limited to these examples.

## Claims

1. A method for preparing chimeric antigen receptor (CAR)-carrying exosomes derived from immune cells wherein the immune cells are T cells or NK cells, comprising the following steps:
A) preparing the CAR expressing immune cells by a general bioengineering technology;
B) antigen-specific activation of the CAR expressing immune cells:
soluble recombinant specific targets, engineered cells expressing the specific targets, or tumor cells expressing the specific targets are adopted as activating agents; wherein the specific targets are antigen targets targeted by a single-chain fragment variable (scFv) expressed by the CAR expressing immune
cells; adding an antigen protein or an immobilized antigen protein to an *in vitro* culture system, directly co-cultivating the CAR expressing immune cells with inactivated engineered cells expressing the specific targets, or with
inactivated tumor cells expressing the specific targets, to obtain antigen-specific activated CAR expressing immune cells;
C) isolation of exosomes of the CAR expressing immune cells:
collecting a culture supernatant of the antigen-specific activated CAR expressing immune cells, and isolating the exosomes by a general exosome isolation method to obtain an exosome suspension;
D) adding magnetic beads coated with a specific antigen (namely, CAR-capturing magnetic beads) to the exosome suspension obtained in step C, wherein, the magnetic beads comprise a recombinant target protein antigen that can specifically bind to the CAR protein; incubating a resulting suspension, and then placing the suspension in a magnetic field; removing the supernatant, and then adding a buffer; adding the resulting suspension to a column, eluting the exosomes retained on the column with buffer, wherein, after the supernatant is added to the column, substances flowing out first are exosomes without the antigen binding ability, and then the column is rinsed with buffer to obtain the CAR-carrying exosomes derived from immune cells that have the antigen binding ability; depending on a volume of an initially collected medium, adding PBS as appropriate to resuspend exosomes; detecting a total protein concentration with Bradford kit; and dispensing and storing the resulting exosomes at -80°C.

2. The method according to claim 1, wherein the immune cells are derived from a patient or a healthy donor.

3. The method according to claim 1, wherein, the immune
cells for preparing the CAR expressing
immune cells in step A are T cells or T cell progenitors, the immune cells are derived from a healthy donor, and the CAR expressing immune cells are prepared by a method comprising the following steps:
(a)activating a an isolated cell sample comprising the T
cells or the T cell progenitors;
(b) constructing viral vectors for scFv-CD8 hinge and TM-4-1BB-CD3, scFv-hinge-TM-CD28-CD3, and scFv-hinge-CD28-4-1BB-CD3;
(c) constructing a recombinant plasmid to package a virus;
(d) infecting the T cells or the T cell progenitors with the virus to obtain CAR-T cells; and
(e) culturing and expanding the CAR-T cells *in vitro.*

4. The method according to claim 1, wherein, in step B, the antigen targets targeted by the scFv in the CAR expressing immune cells are at least one selected from the group consisting of EGFR, HER2, and CD20.

5. The method according to claim 1, wherein, the activating agent used in step B is one selected from the group consisting of an epidermal growth factor receptor (EGFR) extracellular domain recombinant protein, an EGFR extracellular domain recombinant protein cross-linked with magnetic beads, CHO cells expressing EGFR, MDA-MB-231 cells expressing EGFR, an HER2 extracellular domain recombinant protein cross-linked with magnetic beads, BT474 cells expressing HER2, and Raji cells expressing CD20.

6. The method according to claim 1, wherein, the isolation of exosomes derived from CAR expressing immune cells in step C is conducted as follows: centrifuging the collected culture supernatant at 4°C and 2,000 g for 10 min to remove dead cells and large debris; carefully transferring the resulting supernatant to a new sterile centrifuge tube, and then centrifuging at 4°C and 10,000 g for 30 min to remove organelles and small particles; carefully transferring the resulting supernatant to a sterile ultracentrifuge tube, and ultracentrifuging at 4°C and 110,000 g for 70 min; carefully discarding the supernatant, and washing the precipitates with PBS once; and ultracentrifuging the resulting suspension at 4°C and 110,000 g for 70 min to obtain precipitates, namely, exosomes.

7. CAR-carrying exosomes derived from antigen-specific activated immune cells prepared by the preparation method according to any one of claims 1 to 6, wherein the immune cells are T cells or NK cells activated with antigen , and wherein the CAR-carrying exosomes derived from the immune cells carry CAR proteins and a range of the average diameter of the CAR-carrying exosomes derived from the immune cells is 30 nm to 150 nm.

8. A process of preparing anti-tumor drugs or drugs for treating severe infectious diseases or autoimmune diseases, the process comprising using the CAR-carrying exosomes derived from the immune cells according to claim 7 to make a composition thereof.

9. A preparation, wherein, the preparation is a composition comprising the CAR-carrying exosomes derived from the immune cells according to claim 7.

10. CAR-carrying exosomes derived from immune cells according to claim 7 for use as an anti-tumor drug.

## Patentansprüche

1. Verfahren zum Herstellen von aus Immunzellen abgeleiteten Exosomen, die einen heterozygoten Antigenrezeptor (CAR) tragen, wobei die Immunzellen T-Zellen oder NK-Zellen sind, folgende Schritte umfassend:
A) Herstellen der CAR exprimierenden Immunzellen durch allgemeine Biotechnologie;
B) antigenspezifisches Aktivieren der CAR exprimierenden Immunzellen:
wobei lösliche rekombinante spezifische Ziele, veränderte Zellen, die die spezifischen Ziele exprimieren, oder Tumorzellen, die die spezifischen Ziele exprimieren, als Aktivierungsmittel angewandt werden; wobei die spezifischen Ziele Antigenziele sind, die Ziele einer einzelkettigen Fragmentvariablen (scFv) sind, die durch die CAR exprimierenden Immunzellen exprimiert wird; Zusetzen eines Antigenproteins oder eines immobilisierten Antigenproteins zu einem In-vitro-Kultursystem, direktes Co-Kultivieren der CAR exprimierenden Immunzellen mit inaktivierten technisch veränderten Zellen, die die spezifischen Ziele exprimieren, oder mit inaktivierten Tumorzellen, die die spezifischen Ziele exprimieren, um antigenspezifische aktivierte CAR exprimierende Immunzellen zu erlangen;
C) Isolieren der Exosome der CAR exprimierenden Immunzellen:
wobei ein Kulturüberstand der antigenspezifischen aktivierten CAR exprimierenden Immunzellen gesammelt wird und die Exosome durch ein allgemeines Exosomisolationsverfahren isoliert werden, um eine Exosomsuspension zu erlangen;
D) Zusetzen von Magnetperlen, die mit einem spezifischen Antigen beschichtet sind (nämlich CAR erfassende Magnetperlen), zu der in Schritt C erlangten Exosomsuspension, wobei die Magnetperlen ein rekombinantes Zielproteinantigen umfassen, das spezifisch an das CAR-Protein anbinden kann; Inkubieren einer resultierenden Suspension und dann Anordnen der Suspension in einem Magnetfeld; Entfernen des Überstands und dann Zusetzen eines Puffers; Zusetzen der resultierenden Suspension zu einer Säule, Eluieren der an der Säule zurückgehaltenen Exosome mit Puffer, wobei nach dem Zusetzen des Überstands zu der Säule zuerst ausströmende Stoffe Exosome ohne die Antigenbindungsfähigkeit sind, woraufhin die Säule mit Puffer gespült wird, um die CAR-tragenden Exosome zu erlangen, die von Immunzellen abgeleitet sind, die die Antigenbindungsfähigkeit aufweisen; abhängig von einem Volumen eines zunächst gesammelten Mediums Zusetzen von PBS in geeigneter Menge zum Resuspendieren der Exosome; Ermitteln einer Gesamtproteinkonzentration mit einem Bradford-Kit; und Abgeben und Lagern der resultierenden Exosome bei -80 °C.

2. Verfahren nach Anspruch 1, wobei die Immunzellen von einem Patienten oder einem gesunden Spender abgeleitet werden.

3. Verfahren nach Anspruch 1, wobei die Immunzellen zum Herstellen der CAR exprimierenden Immunzellen in Schritt A T-Zellen oder T-Zellvorläufer sind, wobei die Immunzellen von einem gesunden Spender abgeleitet werden und die CAR exprimierenden Immunzellen durch ein Verfahren hergestellt werden, das die folgenden Schritte umfasst:
(a) Aktivieren einer isolierten Zellprobe, die die T-Zellen oder T-Zellvorläufer umfasst;
(b) Konstruieren viraler Vektoren für scFv-CD8-hinge und TM-4-1BB-CD3, scFv-hinge-TM-CD28-CD3 und scFv-hinge-CD28-4-IBB-CD3;
(c) Konstruieren eines rekombinanten Plasmids zum Verpacken eines Virus;
(d) Infizieren der T-Zellen oder T-Zellvorläufer mit dem Virus, um CAR-T-Zellen zu erlangen; und
(e) Kultivieren und Expandieren der CAR-T-Zellen in vitro.

4. Verfahren nach Anspruch 1, wobei in Schritt B die Antigenziele, die Ziel von scFv in den CAR exprimierenden Immunzellen sind, wenigstens eins sind, ausgewählt aus der Gruppe bestehend aus EGFR, HER2 und CD20.

5. Verfahren nach Anspruch 1, wobei das Aktivierungsmittel, das in Schritt B verwendet wird, eines ist, das ausgewählt ist aus der Gruppe bestehend aus einem rekombinanten Protein der extrazellulären Domäne von epidermalem Wachstumsfaktorrezeptror (EGFR), quervernetzt mit Magnetperlen, CHO-Zellen, die EGFR exprimieren, MDA-MB-231-Zellen, die EGFR exprimieren, einem rekombinanten Protein der extrazellulären Domäne von HER2, quervernetzt mit Magnetperlen, BT474-Zellen, die HER2 exprimieren, und Raji-Zellen, die CD20 exprimieren.

6. Verfahren nach Anspruch 1, wobei die Isolation von Exosomen, die von CAR exprimierenden Immunzellen abgeleitet wurden, in Schritt C wie folgt geschieht: Zentrifugieren des gesammelten Kulturüberstands bei 4 °C und 2.000 g für 10 min zum Entfernen toter Zellen und großer Verunreinigungen; sorgfältiges Übertragen des resultierenden Überstands in ein neues steriles Zentrifugenröhrchen und dann Zentrifugieren bei 4 °C und 10.000 g für 30 min zum Entfernen von Organellen und kleinen Teilchen; sorgfältiges Übertragen des resultierenden Überstands in ein steriles Ultrazentrifugenröhrchen und dann Ultrazentrifugieren bei 4 °C und 110.000 g für 70 min; sorgfältiges Verwerfen des Überstands und einmaliges Waschen der Ausfällungen mit PBS; und Ultrazentrifugieren der resultierenden Suspension bei 4 °C und 110.000 g für 70 min, um Ausfällungen, also Exosome, zu erlangen.

7. CAR-tragende Exosome, die aus antigenspezifischen aktivierten Immunzellen abgeleitet sind, die durch das Herstellungsverfahren nach einem der Ansprüche 1 bis 6 hergestellt wurden, wobei die Immunzellen mit Antigen aktivierte T-Zellen oder NK-Zellen sind, und wobei die aus den Immunzellen abgeleiteten CAR-tragenden Exosome CAR-Proteine tragen und ein Bereich des durchschnittlichen Durchmessers der aus den Immunzellen abgeleiteten CAR-tragenden Exosome 30 nm bis 150 nm beträgt.

8. Verfahren zum Herstellen von Antitumorarzneistoffen oder Arzneistoffen zum Behandeln schwerer Infektionskrankheiten oder Autoimmunkrankheiten, wobei das Verfahren Verwenden der aus den Immunzellen abgeleiteten CAR-tragenden Exosome nach Anspruch 7 umfasst, um eine Zusammensetzung daraus herzustellen.

9. Präparat, wobei das Präparat eine Zusammensetzung ist, die die aus den Immunzellen abgeleiteten CAR-tragenden Exosome nach Anspruch 7 umfasst.

10. Aus Immunzellen abgeleiteten CAR-tragende Exosome nach Anspruch 7 zur Verwendung als Antitumorarzneistoff.

## Revendications

1. Un procédé de préparation d'exosomes porteuses de récepteur d'antigène chimérique (CAR) dérivés de cellules immunes dans lequel les cellules immunes sont des cellules T ou des cellules NK, comprenant les étapes suivantes :
A) préparation des cellules immunes exprimant le CAR par un procédé de technologie de bio-ingénierie générale ;
B) activation spécifique antigène des cellules immunes exprimant le CAR :
des cibles spécifiques recombinantes solubles, des cellules modifiées exprimant les cibles spécifiques, ou des cellules tumorales exprimant les cibles spécifiques sont utilisées comme agents d'activation ; dans laquelle les cibles spécifiques sont des cibles antigènes ciblées par un fragment d'anticorps à chaîne unique (scFv) exprimé par les cellules immunes exprimant le CAR ; ajout d'une protéine antigène ou d'une protéine antigène immobilisée à un système de culture in vitro, co-culture directe des cellules immunes exprimant le CAR avec les cellules modifiées inactivées exprimant les cibles spécifiques, ou avec des cellules tumorales exprimant les cibles spécifiques, pour obtenir les cellules immunes exprimant le CAR à spécificité antigène activée ;
C) isolement des exosomes des cellules immunes exprimant le CAR :
collecte du surnageant de culture des cellules immunes exprimant le CAR à spécificité antigène activée, et isolement des exosomes par un procédé général d'isolement d'exosomes pour obtenir une suspension d'exosome ;
D) ajout de billes magnétiques enduites d'antigène spécifique (nommément, billes magnétiques de capture de CAR) à la suspension d'exosome obtenue à l'étape C, dans lequel, les billes magnétiques comprennent un antigène de protéine cible recombinante capable de se lier spécifiquement à la protéine de CAR ; incubation de la suspension résultante, puis placement de la suspension dans un champ magnétique ; élimination du surnageant, et ajout d'un tampon ; ajout de la suspension résultante sur colonne, élution des exosomes retenues sur la colonne à l'aide du tampon, dans lequel une fois que le surnageant est ajouté à la colonne, les substances qui s'écoulent en premier sont les exosomes sans capacité de liaison antigène, puis la colonne est rincée à l'aide d'un tampon pour obtenir les exosomes porteuses de CAR dérivées des cellules immune qui sont munie de la capacité de liaison antigène ; en fonction du volume du milieu collecté à l'origine, ajout du PBS approprié pour re-suspendre les exosomes ; détection de la concentration de protéines totales à l'aide d'un kit Bradford ; et délivrance et stockage des exosomes résultantes à -80°C.

2. Le procédé selon la revendication 1, dans lequel les cellules immunes sont dérivées d'un patient ou d'un donneur sain.

3. Le procédé selon la revendication 1, dans lequel les cellules immunes pour préparer les cellules immunes exprimant le CAR de l'étape A sont des cellules T ou des progéniteurs de cellules T, les cellules immunes sont dérivées d'un donneur sain, et les cellules immunes exprimant le CAR sont préparées par un procédé comprenant les étapes suivantes :
(a) activation d'un échantillon cellulaire isolé comprenant les cellules T ou les progéniteurs de cellules T ;
(b) construction des vecteurs viraux pour scFv-CD8 hinge et TM-4-1BB-CD3, scFv-hinge-TM-CD28-CD3, et scFv-hinge-CD28-4-IBB-CD3 ;
(c) construction d'un plasmide recombinant pour envelopper un virus ;
(d) infection des cellules T ou des progéniteurs de cellules T avec le virus pour obtenir des cellules CAR-T ; et
(e) culture et expansion des cellules CAR-T in vitro.

4. Le procédé selon la revendication 1, dans lequel à l'étape B, les cibles antigènes ciblées par le scFv dans les cellules immunes exprimant le CAR sont d'au moins une choisie au sein d'un groupe constitué de EGFR, de HER2, et de CD20.

5. Le procédé selon la revendication 1, dans lequel l'agent d'activation utilisé à l'étape B est choisi au sein d'un groupe constitué de la protéine recombinante du domaine extracellulaire du récepteur de facteur de croissance épidermique (EGFR), une protéine recombinante du domaine extracellulaire EGFR réticulée avec des billes magnétiques, des cellules CHO exprimant le EGFR, des cellules MDA-MB-231 exprimant le EGFR, une protéine recombinante du domaine extracellulaire HER2 réticulée avec des billes magnétiques, des cellules BT474 exprimant le HER2, et des cellules Raji exprimant le CD20.

6. Le procédé selon la revendication 1, dans lequel l'isolement des exosomes dérivées des cellules immunes exprimant le CAR à l'étape C est effectué de la façon suivant : centrifugation du surnageant collecté à 4°C et 2,000 g pendant 10 minutes pour éliminer les cellules mortes et les gros débris; transfert avec précaution du surnageant résultant dans un nouveau tube de centrifugation stérile, puis centrifugation à 4°C et 10,000 g pendant 30 minutes pour éliminer les organelles et petites particules ; transferts avec précaution du surnageant résultant dans un tube d'ultracentrifugation stérile, et ultracentrifugation à 4°C et 110,000 g pendant 70 minutes ; rejet avec précaution du surnageant, et lavement du précipité une fois au PBS ; et ultracentrifugation de la suspension résultante à 4°C et 110,000 g pendant 70 minutes pour obtenir le précipité, nommément, les exosomes.

7. Des exosomes porteuses de CAR dérivées de cellules immunes à spécificité antigène activée préparées à l'aide du procédé de préparation selon l'une quelconque des revendications 1 à 6, dans lesquels les cellules immunes sont des cellules T ou des cellules NK activées par antigène, et dans lesquels les exosomes porteuses de CAR dérivées des cellules immunes portant des protéines CAR et l'amplitude de diamètre moyen des exosomes porteuses de CAR dérivées des cellules immunes sont de 30 nm à 150 nm.

8. Un procédé de préparation de médicament anti-tumoral ou des médicaments pour traiter les maladies infectieuses graves ou les maladies auto-immunes, le procédé comprenant l'utilisation des exosomes porteuses de CAR dérivées des cellules immunes selon la revendication 7 pour en faire la composition.

9. Une préparation, dans laquelle, la préparation est une composition comprenant les exosomes porteuses de CAR dérivées des cellules immunes selon la revendication 7.

10. Les exosomes porteuses de CAR dérivées des cellules immunes selon la revendication 7 pour une utilisation en tant que médicament anti-tumoral.
